# EUROPEAN PATENT APPLICATION

(11) **EP 4 410 278 A1**
(43) Date of publication of application: **07.08.2024**
(21) Application number: 22876888.3
(22) Date of filing: 29.09.2022
(51) Int. Cl.: A61K 9/20, A61K 31/7048, A61P 3/10

(54) **PHARMACEUTICAL COMPOSITION COMPRISING ENAVOGLIFLOZIN**

(30) Priority: 30.09.2021 KR 20210130239
(71) Applicant: Daewoong Pharmaceutical Co., Ltd., Hwaseong-si, Gyenoggi-do 18623 (KR)
(72) Inventor: HA, Songyi, Yongin-si Gyeonggi-do 16863 (KR); KIM, Gyoungwon, Yongin-si Gyeonggi-do 17009 (KR); KIM, Gwanyoung, Yongin-si Gyeonggi-do 16824 (KR); CHO, Sangeun, Yongin-si Gyeonggi-do 16899 (KR); HWANG, On, Suwon-si Gyeonggi-do 16593 (KR); PARK, Minhyung, Suwon-si Gyeonggi-do 16587 (KR); LEE, Seoyeo, Suwon-si Gyeonggi-do 16675 (KR); LEE, Heewon, Yongin-si Gyeonggi-do 16908 (KR); YOUN, Seungbin, Suwon-si Gyeonggi-do 16509 (KR)
(74) Representative: Plasseraud IP
(86) International application number: PCT/KR2022/014640
(87) International publication number: WO 2023/055116

(57) **Abstract**

The present invention relates to a pharmaceutical composition comprising enavogliflozin, which is a selective inhibitor of sodium-glucose cotransporter 2. A pharmaceutical composition comprising a compound of Chemical Formula 1 according to the present invention enables implementation of a formulation having excellent content uniformity, formulation uniformity, elution profile, and the like, despite comprising a low dose of a drug.

## Description

### [Technical Field]

The present invention relates to a pharmaceutical composition including enavogliflozin, which is a selective inhibitor of sodium-glucose cotransporter 2.

### [Background Art]

Sodium-glucose cotransporter 2 (SGLT2) inhibitors belong to a new class of antihyperglycemic drugs. SGLT-2 inhibitors reduce glucose reabsorption in the proximal nephron to increase glucose excretion through mechanisms independent of insulin, and the safety and efficacy of the SGLT2 inhibitors for the treatment of type II diabetes have been confirmed through many studies.

In particular, U.S. Patent Publication No. 2015/0152075 discloses enavogliflozin of the following Chemical Formula 1 as a compound having a diphenylmethane residue that exhibits inhibitory activity against SGLT2. Also, the document discloses that enavogliflozin has an excellent inhibitory effect on human SGLT2 activity, and thus is effective in treating diabetes.

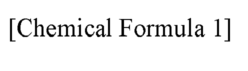

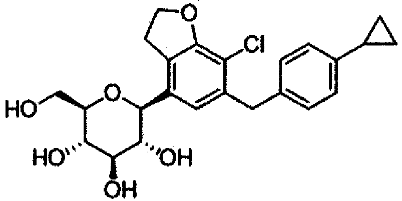

Compound Name: (2S,3R,4R,5S,6R)-2-(7-chloro-6-(4-cyclopropylbenzyl)-2,3-dihydrobenzofuran-4-yl)-6-(hydroxymethyl)tetrahydro-2H-pyran-3,4,5-triol

Enavogliflozin is a drug that is currently in phase 3 clinical trials. As a result of phase 2 clinical trials, it was confirmed that all the groups administered enavogliflozin at concentrations of 0.1 mg, 0.3 mg, and 0.5 mg have a statistically significant blood sugar-lowering effect compared to the placebo.

It is evaluated that enavogliflozin shows excellent urinary sugar secretion (glucose excreted in urine) efficacy at a very low dose of 1/100^{th} of the dose compared to the same class of drugs.

### [Disclosure]

### [Technical Problem]

As described above, although enavogliflozin has been shown to have an excellent blood sugar-lowering effect even at very low doses, it has been found that problems such as dissolution profile, content uniformity, and formulation uniformity, which are caused when manufacturing a pharmaceutical composition including a small content of an active ingredient, must be resolved.

### [Technical Solution]

The present inventors have conducted various studies on the formulation of enavogliflozin, and found that problems such as dissolution profile, content uniformity, formulation uniformity, and the like of an enavogliflozin-containing preparation can be resolved when the pharmaceutical composition is composed as follows.

Specifically, the present invention provides a pharmaceutical composition including a compound of Chemical Formula 1 or a pharmaceutically acceptable salt thereof, which serves as an active ingredient, an excipient, a disintegrant, and a binder, wherein the compound of Chemical Formula 1 has an average particle size of 15 µm or less.

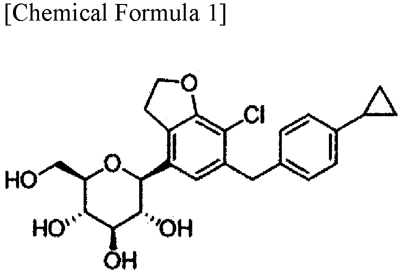

Enavogliflozin is preferably formulated in an immediate-release dosage form due to the nature of the drug used as an anti-diabetic agent. However, according to the following embodiments, it was confirmed that the dissolution profile of the drug significantly changes depending on the average particle size of enavogliflozin.

According to an exemplary embodiment of the present invention, enavogliflozin may have an average particle size of 15 µm or less, preferably 10 µm or less.

When the average particle size of enavogliflozin is greater than 15 µm, the 5-minute dissolution rate is very low, that is, less than 40% of the total content of enavogliflozin, and the 30-minute dissolution rate is less than 80%, which results in an inadequate final dissolution rate.

When microionization of the drug particle size is required, particles may be pulverized using a conventional mill capable of pulverizing particles, such as a jet mill, a hammer mill, a ball mill, a fluid energy mill, or the like. Also, the particle size of the drug may be subdivided using a size classification method such as a sieving method performed using a sieve, air current classification, or the like. Methods for controlling a desired particle size are well known in the art. See, for example, the following literature: [Pharmaceutical dosage forms: volume 2, 2nd edition, Ed.: H. A. Lieberman, L. Lachman, J. B. Schwartz (Chapter 3: SIZE REDUCTION)].

In this specification, the particle size of the drug is expressed based on the particle size distribution as D(X) = Y (where X and Y are positive numbers). D(X) = Y means that the particle diameter at a point where the particle size of a drug reaches X% (% is calculated based on number, volume, or weight) by accumulating the particle size of the drug in descending order is Y when the particle size distribution of any drug obtained by measuring the particle diameter of the drug in a formulation is represented by a cumulative curve. For example, D(10) represents a diameter of particles at the point where the particle size of the drug reaches 10% by accumulating the particle size of the drug in descending order, D(50) represents a diameter of particles at the point where the particle size of the drug reaches 50% by accumulating the particle size of the drug in descending order, and D(90) represents a diameter of particles at the point where the particle size of the drug reaches 90% by accumulating the particle size of the drug in descending order.

Whether the particle size distribution (D(X)) represents a percentage of the total accumulated particles based on any one of number, volume, or weight depends on the method used to measure the particle size distribution. Methods of measuring the particle size distribution and the types of percentages (%) associated therewith are known in the art. For example, when the particle size distribution is measured using the well-known laser diffraction method, the value of X in D(X) represents the percentage calculated by volume average. It is well known to those skilled in the art that the results of particle size distribution measurement obtained by a particular method may be correlated with those obtained from other techniques based on experience through routine experimentation. For example, because the laser diffraction method is sensitive to the volume of particles, it provides a volume average particle size, which corresponds to a weight average particle size when the density is constant.

In the present invention, the particle size distribution of drug particles may be measured using a commercially available device according to a laser diffraction/scattering method based on the Mie theory. For example, measurements are performed using a commercially available device such as a Mastersizer laser diffraction device (Malvern Instruments). In this device, when particles are irradiated with a helium-neon laser beam and a blue light-emitting diode, scattering occurs, and a light scattering pattern appears on a detector. By analyzing such a light scattering pattern according to the Mie theory, the particle diameter distribution is obtained. The measurement method may be either dry or wet.

For reference, according to an embodiment of the present invention, the particle size of the drug is measured by laser diffraction using the volume average particle size.

According to an exemplary embodiment of the present invention, the compound of Chemical Formula 1 may be included in an amount of less than 1 part by weight based on 100 parts by weight of the total pharmaceutical composition.

As the optimal once-daily dose of enavogliflozin determined during clinical trials ranges from 0.1 to 0.5 mg, when the pharmaceutical composition is formulated as a unit dosage form, the content of the active ingredient in the pharmaceutical composition ranges from 0.1 to 0.5 mg.

The pharmaceutical composition according to the present invention includes pharmaceutically acceptable additives in addition to the compound of Chemical Formula 1, which is the active ingredient.

The pharmaceutical composition of the present invention includes an excipient, a disintegrant, a binder, and the like as the additives.

Examples of excipients include lactose (including hydrates), dextrin, mannitol, sorbitol, starch, microcrystalline cellulose (*e.g*., Celphere^{™}), silicified microcrystalline cellulose (*e.g*., Prosolve^{™}), calcium phosphate hydrate, anhydrous calcium phosphate, calcium carbonate, sugars, or mixtures thereof. In an exemplary embodiment of the present invention, the preferred excipient is microcrystalline cellulose.

Examples of disintegrants include crospovidone, croscarmellose sodium, sodium starch glycolate, and low-substituted hydroxypropyl cellulose. In an exemplary embodiment of the present invention, the preferred excipient is croscarmellose sodium.

Examples of binders include polyvinylpyrrolidone, povidone, gelatin, starch, sucrose, methyl cellulose, ethyl cellulose, hydroxyethyl cellulose, hydroxypropyl cellulose, hydroxypropylalkyl cellulose (*e.g.*, hydroxypropylmethyl cellulose), and mixtures thereof. In an exemplary embodiment of the present invention, the preferred binder is hydroxypropyl cellulose.

Examples of other additives include a lubricant, a colorant, and the like.

The lubricant includes stearic acid, stearate (*e.g.*, magnesium stearate), light anhydrous silicic acid, talc, corn starch, carnauba wax, magnesium silicate, synthetic aluminum silicate, hydrogenated oil, white beeswax, titanium oxide, microcrystalline cellulose, Macrogol 4000, and 6000, isopropyl myristate, calcium hydrogen phosphate, and mixtures thereof.

According to an exemplary embodiment of the present invention, the excipient may be included in an amount of 80 to 95 parts by weight based on 100 parts by weight of the total pharmaceutical composition.

According to an exemplary embodiment of the present invention, the disintegrant may be included in an amount of 2 to 8 parts by weight based on 100 parts by weight of the total pharmaceutical composition. When the content of the disintegrant is less than 2 parts by weight based on 100 parts by weight of the total pharmaceutical composition, the dissolution rate may be delayed due to low initial disintegration power, which may affect Cₘₐₓ in the body. Also, when the content of the disintegrant is greater than 8 parts by weight based on 100 parts by weight of the total pharmaceutical composition, the overall flowability of the granules may be deteriorated due to the increased amount of the disintegrant in the post-mixed part.

According to an exemplary embodiment of the present invention, the binder may be included in an amount of 3 to 10 parts by weight based on 100 parts by weight of the total pharmaceutical composition. When the content of the binder is less than 3 parts by weight based on 100 parts by weight of the total pharmaceutical composition, it may be difficult to form and maintain suitable dry granules, which may affect the maintenance of homogeneous dispersibility of the main ingredients and the flowability of the granules due to the generation of fine powder. Also, when the content of the binder is greater than 10 parts by weight based on 100 parts by weight of the total pharmaceutical composition, a granular material having a strong binding force is formed, which affects the solubility of the initially disintegrated granule particles upon dissolution, which may eventually affect Cₘₐₓ in the body.

The pharmaceutical composition according to the present invention corresponds to an immediate-release formulation.

According to one exemplary embodiment of the present invention, the dissolution rate of the pharmaceutical composition may be 50% or more, preferably 60% or more of the total content of the active ingredient after 5 minutes.

According to one exemplary embodiment of the present invention, the dissolution rate of the pharmaceutical composition may be 80% or more, preferably 85% or more of the total content of the active ingredient after 15 minutes.

According to one exemplary embodiment of the present invention, the dissolution rate of the pharmaceutical composition may be 85% or more, preferably 90% or more of the total content of the active ingredient after 30 minutes.

Conversely, because the dissolution rate of the active ingredient in the pharmaceutical composition affects the maximum blood concentration (Cₘₐₓ) and the area under the blood concentration-time curve (AUC) when administering the drug, it is important to adjust the dissolution rate of the pharmaceutical composition in order to achieve the appropriate Cₘₐₓ and AUC. Because enavogliflozin has a Tₘₐₓ of 1 to 2 hours, the drug absorption rate in the stomach is considered important. The dissolution rate is measured under the conditions of a pH 1.2 dissolution medium by the Korean Pharmacopoeia Dissolution Test Method 2 (a paddle method). For specific conditions, see the experimental examples below.

Also, the present invention provides a pharmaceutical composition including a granular material in which pre-mixed granules and a post-mixed part are mixed, wherein the pre-mixed granules include a compound of Chemical Formula 1 or a pharmaceutically acceptable salt thereof.

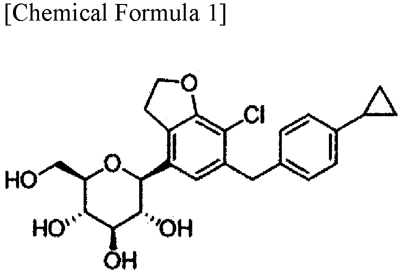

In the course of research on the formulation of the compound of Chemical Formula 1, the present inventors confirmed that preparing a granular material and formulating the granular material into tablets and the like is advantageous in terms of drug content uniformity and formulation uniformity.

In the pharmaceutical composition, the granular material is prepared by mixing the pre-mixed granules with the post-mixed part.

The pre-mixed granules may include the compound of Chemical Formula 1 or a pharmaceutically acceptable salt thereof, an excipient, a binder, and a lubricant.

Also, the post-mixed part may include an excipient, a disintegrant, and a lubricant.

The excipient, binder, disintegrant, lubricant, and the like are the same as those described above, and thus descriptions thereof are omitted to avoid redundant description.

According to an exemplary embodiment of the present invention, each of the pre-mixed granules and the post-mixed part may include an excipient. More specifically, each of the pre-mixed granules and the post-mixed part may include microcrystalline cellulose as the excipient.

According to the following embodiments, it is found that the microcrystalline cellulose included in the pre-mixed granules and the post-mixed part affects the content uniformity of the drug depending on the particle size and bulk density thereof.

According to an exemplary embodiment of the present invention, the microcrystalline cellulose in the pre-mixed granules may have a particle size of 130 µm or less, preferably 60 to 130 µm. The microcrystalline cellulose in the pre-mixed granules may have a bulk density of 0.26 to 0.33. When the particle size and bulk density of the microcrystalline cellulose in the pre-mixed granules satisfy the above conditions, a formulation having a low standard deviation (SD) of content uniformity may be obtained. When the particle size of the microcrystalline cellulose in the pre-mixed granules is 130 um or less, the content uniformity of the pre-mixed granules, the content uniformity of the final granules, and formulation uniformity are all good, and the Carr's index value, which indicates the physical properties of the final granules, is also good, confirming that the flowability of the formulation is also excellent. On the other hand, when the particle size of the microcrystalline cellulose in the pre-mixed granules is greater than 130 µm, it is confirmed that both the content uniformity of the pre-mixed granules and the content uniformity of the final granules are not suitable due to their large deviations, and formulation uniformity is also poor.

Meanwhile, the microcrystalline cellulose in the post-mixed part may have a particle size of 130 µm or more, preferably 130 to 250 µm. The excipient in the post-mixed part may have a bulk density of 0.28 to 0.37. When the particle size of the microcrystalline cellulose in the post-mixed part is less than 130 µm, it can be seen that the Carr's index value, which indicates the physical properties of the final granules, is not appropriate, and granule flowability becomes weak.

When the microcrystalline cellulose in the pre-mixed granules is relatively compared to the microcrystalline cellulose in the post-mixed part, it is confirmed that it is desirable for the microcrystalline cellulose included in the pre-mixed granules to have a small particle size, whereas it is desirable that the particle size of the microcrystalline cellulose in the post-mixed part is relatively larger than the particle size of the microcrystalline cellulose included in the pre-mixed granules.

According to the following embodiments, it is confirmed that not only the particle sizes of the microcrystalline cellulose in the pre-mixed granules and the microcrystalline cellulose in the post-mixed part, but also the weight ratio of the excipient in the pre-mixed granules and the excipient in the post-mixed part affect the content uniformity of the drug.

According to an exemplary embodiment of the present invention, the weight ratio of the excipient in the pre-mixed granules and the excipient in the post-mixed part may range from 4:1 to 1:1. As the proportion of the microcrystalline cellulose in the post-mixed part increases, the flowability of the granules becomes better, while the content deviation of the granules increases. Therefore, it is found that it is desirable to adjust the weight ratio within the above appropriate range.

Meanwhile, in the pharmaceutical composition according to the present invention, the binder may be one or more selected from the group consisting of hydroxypropyl cellulose, povidone, copovidone, and hypromellose.

According to one exemplary embodiment of the present invention, the binder may be hydroxypropyl cellulose, which may have a weight average molecular weight of less than 200,000. When hydroxypropyl cellulose having a weight average molecular weight of 200,000 or more is used, both the 5-minute dissolution rate and the 30-minute dissolution rate are low, which is undesirable in terms of bioavailability.

Meanwhile, with regard to Carr's index, which is used as a measure of flowability in formulation, the Carr's index of the granular material preferably ranges from 21 to 25.

In the pharmaceutical composition of the present invention, the granular material may be a dry granular material, but the present invention is not limited thereto. According to another exemplary embodiment, the granular material may be a wet granular material.

In the present invention, the pharmaceutical composition may have a dosage form for oral administration such as tablets, capsules, and the like. According to one exemplary embodiment of the present invention, the pharmaceutical composition may be in the form of a tablet.

According to preferred embodiments, the pharmaceutical composition may include the compound of Chemical Formula 1 in a dose of 0.3 mg.

The pharmaceutical composition according to the present invention may be administered orally once a day, but the present invention is not limited thereto.

### [Advantageous Effects]

A pharmaceutical composition including a compound of Chemical Formula 1 according to the present invention enables implementation of a formulation having excellent content uniformity, formulation uniformity, dissolution profile, and the like, although the pharmaceutical composition includes a low dose of a drug.

### [Description of Drawings]

FIG. 1 shows the results of evaluating the dissolution rates of tablets prepared in Example 1, Example 2, Example 3, Comparative Example 1, and Comparative Example 2 (dissolution medium: pH 1.2).
FIG. 2 shows the results of evaluating the dissolution rates of the tablets prepared in Example 1, Example 2, Example 3, Comparative Example 1, and Comparative Example 2 (dissolution medium: pH 4.0).
FIG. 3 shows the results of evaluating the dissolution rates of the tablets prepared in Example 1, Example 2, Example 3, Comparative Example 1, and Comparative Example 2 (dissolution medium: pH 6.8).
FIG. 4 shows the results of evaluating the dissolution rates of the tablets prepared in Example 1, Example 2, Example 3, Comparative Example 1, and Comparative Example 2 (dissolution medium: DW).
FIG. 5 shows the results of evaluating the dissolution rates of tablets prepared in Example 1, Example 8, and Comparative Example 6 (dissolution medium: pH 1.2).

### [Mode for Invention]

Hereinafter, preferred embodiments of the present invention are presented in order to aid in understanding the present invention. However, it should be understood that the following embodiments are given by way of illustration only to more easily understand the present invention, and are not intended to limit the present invention.

### Preparation of tablets according to particle size of enavogliflozin

Enavogliflozin having various particle sizes was prepared according to a conventional method, and the particle size of the prepared raw drug was measured as follows.

### Particle size test (Equipment: Malvern's Mastersizer 3000)

### 1) Test solution

0.05% (v/v) lecithin in hexane solution

### 2) Preparation of sample solution

About 10.0 mg of this drug was taken and placed in a 20 mL beaker, and 15 mL of the test solution was added thereto. The resulting mixture was completely dispersed by sonication for 30 seconds, and used as a sample solution.

### 3) Analysis method

The sample solution was added until the obscuration level reached 5% to 10%, and it was then confirmed that the obscuration level was stabilized. Thereafter, measurements were performed as follows:

### [Operating conditions]

Range: 0.02 to 2000 µm
Particle RI: 1.59
Absorption: 0.01
Dispersant RI: 1.380
Obscuration range: 5 to 10%
Stirrer/pump speed: 3000 RPM
Ultrasonic sound: off
Measurement cycles: 5

Immediate-release formulations were prepared using enavogliflozin having various particle sizes through a dry granulation process according to the following steps.
Step 1: 0.3 g of enavogliflozin, 50.0 g of microcrystalline cellulose (PH-102), 4.0 g of hydroxypropyl cellulose (HPC-L), 1.0 g of light anhydrous silicic acid, and 0.5 g of magnesium stearate were mixed.
Step 2: The mixture of Step 1 was prepared into a plate-shaped compressed product using a dry granulator, and pulverized using Comil to prepare a dry granular material.
Step 3:
   15.7 g of microcrystalline cellulose (Vivapur12), 3.0 g of croscarmellose sodium, and 0.5 g of magnesium stearate, which are the post-mixed part, were added to the pre-mixed granules of Step 2, and mixed.
Step 4: The mixed granular material of Step 3 was compression-molded to a total weight of 75.0 mg to prepare tablets.

**[Table 1]**

| | Enavogliflozin | | | Type and amount of additive (mg/T) |
|---|---|---|---|---|
| | D50 (µm) | D90 (µm) | Dₘₑₐₙ | microcrystalline cellulose 65.7 |
| Example 1 | 1.0 | 3.0 | 1.0 | croscarmellose 3.0 |
| Example 2 | 1.0 | 5.0 | 2.0 | hydroxylpropyl cellulose 4.0 |
| Example 3 | 5.0 | 21.0 | 8.0 | light anhydrous silicic acid 1.0 |
| Comparative Example 1 | 14.0 | 45.0 | 19.0 | magnesium stearate 1.0 |
| Comparative Example 2 | 37.0 | 207.0 | 75.0 | |

| | | | | |
|---|---|---|---|---|
| D50 = Median diameter of particle distribution | | | | |

### Experimental Example 1: Dissolution test of tablets according to particle size of enavogliflozin

A dissolution test was performed on the tablets prepared in Example 1, Example 2, Example 3, Comparative Example 1, and Comparative Example 2 according to the following methods and conditions in order to check the difference in dissolution rate depending on the particle size.
1) Dissolution method: Korean Pharmacopoeia Dissolution Method 2 (paddle method)
2) Dissolution medium: pH 1.2/pH 4.0/pH 6.8/DW
3) Dissolution medium amount: 500 mL
4) Dissolution vessel temperature: 37.5°C ± 0.5°C
5) Paddle speed: 50 rpm
6) Analysis method: HPLC method
   - Detector: UV absorptiometer (Measurement wavelength: 225 nm)
   - Column: C18 5 µm/4.6 mm x 150 mm column
   - Mobile phase: hydrogen phosphate buffer + acetonitrile

As a result, in all four solutions, the dissolution rates of Examples 1 to 3 using raw materials having an average particle size (Dₘₑₐₙ) of 10 µm or less exhibited a similar pattern. Because enavogliflozin is a component having a Tₘₐₓ of approximately 1 hour, dissolution in gastric juice is expected to have a significant influence on bioavailability. Therefore, it was judged that the dissolution rate at pH 1.2 was particularly important, and the dissolution rates of Examples 1 to 3 were judged to be appropriate due to the characteristics of enavogliflozin, which is preferably formulated as an immediate-release dosage form. Specifically, the overall dissolution rates of Examples 2 and 3 were similar to that of Example 1, and the similarity factor values also showed an equality of 50% or more.

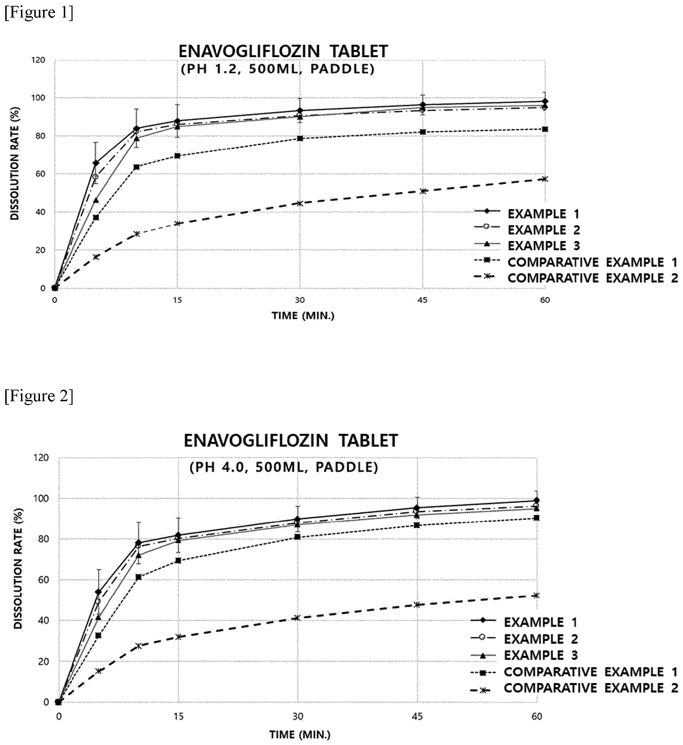

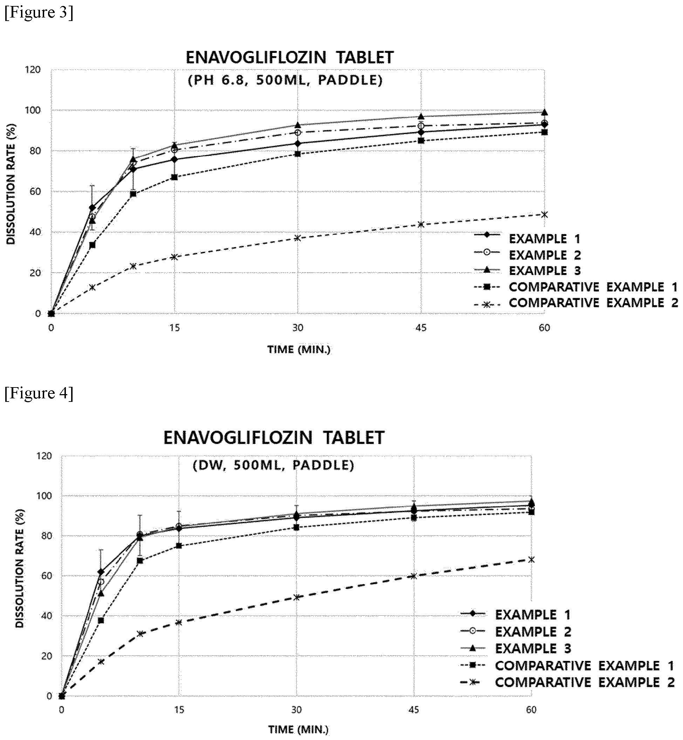

The tablets of Comparative Examples 1 and 2, which had an average raw material particle size of 19 µm or more, showed decreased dissolution rates compared to that of Example 1, and the similarity factor values were also less than 50%, which was significantly different. Therefore, when the average raw material particle size of enavogliflozin was set to 15 µm or less, preferably the average raw material particle size of enavogliflozin was set to 10 µm or less as in Examples 1 to 3, it was judged that the uniform quality and effectiveness of the products in the body can be secured. The results of dissolution rate evaluation are shown in detail in Table 2 below and FIGS. 1 to 4.

**[Table 2]**

| Dissolution medium pH 1.2 | | | | | | |
|---|---|---|---|---|---|---|
| | 5-minute dissolution rate (%) | | 15-minute dissolution rate (%) | | 30-minute dissolution rate (%) | |
| | Average | Standard deviation | Average | Standard deviation | Average | Standard deviation |
| Example 1 | 65.7 | 10.9 | 87.9 | 8.5 | 93.3 | 6.3 |
| Example 2 | 58.3 | 2.4 | 86.1 | 0.9. | 90.7 | 3.8 |
| Example 3 | 46.5 | 3.0 | 84.9 | 0.8 | 90.1 | 3.0 |
| Comparative Example 1 | 37.2 | 1.4 | 69.4 | 5.6 | 78.7 | 2.2 |
| Comparative Example 2 | 16.4 | 1.8 | 33.8 | 3.1 | 44.7 | 4.8 |

### Preparation of granular material according to particle size distribution of excipient in pre-mixed granules

In order to ensure content uniformity and formulation uniformity in the formulation of enavogliflozin, a granular material in which pre-mixed granules and a post-mixed part were mixed was prepared. First, the most appropriate particle size of the excipient for the pre-mixed granules was searched for.

In Example 1 prepared previously, tablets of Example 4 and Comparative Example 3 were prepared in the same manner as in Example 1, except that the particle size and bulk density of the microcrystalline cellulose used in the pre-mixed granules were different (see Table 4). The compositions of Example 1, Example 4, and Comparative Example 3 are shown in Table 3 below.

**[Table 3]**

| **Process** | **Additive** | **Example 1** | **Example 4** | **Comparative Example 3** |
|---|---|---|---|---|
| Pre-mixed granules | Enavogliflozin | 0.3 | 0.3 | 0.3 |
| | Microcrystalline cellulose (Vivapur 102) | 50.0 | | |
| | Microcrystalline cellulose (Vivapur 101) | | 50.0 | |
| | Microcrystalline cellulose (Vivapur12) | | | 50.0 |
| | Hydroxypropyl cellulose | 4.0 | 4.0 | 4.0 |
| | Light anhydrous silicic acid | 1.0 | 1.0 | 1.0 |
| | Magnesium stearate | 0.5 | 0.5 | 0.5 |
| Post-mixed part | Microcrystalline cellulose (Vivapur12) | 15.7 | 15.7 | 15.7 |
| | Croscarmellose sodium | 3.0 | 3.0 | 3.0 |
| | Magnesium stearate | 0.5 | 0.5 | 0.5 |
| Total | | 75 | 75 | 75 |

**[Table 4]**

| **Classification** | **D50** | **Bulk density (g/mL)** |
|---|---|---|
| Vivapur 101 | 65 µm | 0.26 - 0.31 |
| Vivapur 102 | 130 µm | 0.28 - 0.33 |
| Vivapur 12 | 180 µm | 0.30 - 0.36 |
| Vivapur 200 | 250 µm | 0.31 - 0.37 |

### Experimental Example 2: Evaluation of content uniformity of pre-mixed granules and final granules

The content uniformity of the pre-mixed granules and the final granules was evaluated for the tablets prepared in Example 1, Example 4, and Comparative Example 3. The results are shown in Table 5 below. In this case, the content uniformity evaluation was performed in the following manner.
1) Content test method: 1.5 g of the granules to be measured are taken, and placed in a 100 mL volumetric flask, and 50 mL of an extract is added thereto. The resulting mixture is completely dispersed by ultrasonic extraction for 20 minutes. Thereafter, the mixture is stirred for 30 minutes, and cooled sufficiently to room temperature, and an extract is added to adjust to the gauge mark. An appropriate amount of this solution is taken, and centrifuged at 3000 rpm for 10 minutes, and the supernatant is filtered through a 0.45 µm RC membrane filter. Then, the first 2 mL of the filtered solution is discarded, and the filtrate is used as the sample solution.
2) Extract: 1.36 g of potassium dihydrogen phosphate is accurately weighed, and dissolved in 1000 mL of water, and the pH is adjusted to 3.0 using phosphoric acid.
3) Temperature condition: maintained at 35°C ± 0.5°C
4) Analysis method: HPLC method
   - Detector: UV absorptiometer (Measurement wavelength: 225 nm)
   - Column: C18 5 µm/4.6 mm x 150 mm column
   - Mobile phase: hydrogen phosphate buffer + acetonitrile

The content uniformity of the pre-mixed granules and the final granules of Example 1, Example 4, and Comparative Example 3 was tested using the above method. The results are summarized in detail in Table 5.

Because the main ingredient, enavogliflozin, is distributed in a very small amount (less than 0.5%) in the tablet and the average particle size is small, the average particle size of the microcrystalline cellulose used in the pre-mixed granules is expected to have a great influence on the content uniformity of the main ingredient granules and the tablets.

The content uniformity was checked by varying the average particle size of the microcrystalline cellulose used in the pre-mixed granules. As a result, all of Examples 1 to 4 were judged to be suitable. In Comparative Example 3, in which the average particle size of microcrystalline cellulose was 180 µm or more, it was confirmed that the content of enavogliflozin in the granules and the tablets was noticeably non-uniform compared to that of Example 1. Therefore, when the average particle size (D50) and the bulk density of the microcrystalline cellulose used in the pre-mixed granules are set to 130 µm or less and 0.28 to 0.33, respectively (Example 1), it judged that it is possible to prepare tablets with equal quality.

**[Table 5]**

| | | **Example 1** | **Example 4** | **Comparative Example 3** |
|---|---|---|---|---|
| Content uniformity of pre-mixed granules | Average (%) | 97.0 | 102.8 | 101.4 |
| | SD | 1.2 | 1.8 | 5.5 |
| Content uniformity of | Average (%) | 96.3 | 100.3 | 101.0 |
| final granules | SD | 0.7 | 1.8 | 5.2 |
| Formulation uniformity | Acceptance value (%) | 4.5 | 3.4 | 10.4 |

### Preparation of granular material according to particle size distribution of excipient in post-mixed part

The particle size of the excipient in the pre-mixed granules was searched for, and the most appropriate particle size of the excipient for the post-mixed part was searched for.

Tablets of Comparative Example 4 and Example 5 were prepared in the same manner as in Example 1, except that the particle size distribution of the microcrystalline cellulose used in the post-mixed part was changed as shown in Table 6 below.

**[Table 6]**

| **Process** | **Additive** | **Comparative Example 4** | **Example 5** |
|---|---|---|---|
| Pre-mixed granules | Enavogliflozin | 0.3 | 0.3 |
| | Microcrystalline cellulose (Vivapur 102) | 50.0 | 50.0 |
| | Hydroxypropyl cellulose | 4.0 | 4.0 |
| | Light anhydrous silicic acid | 1.0 | 1.0 |
| | Magnesium stearate | 0.5 | 0.5 |
| Post-mixed part | Microcrystalline cellulose (Vivapur 101) | 15.7 | |
| | Microcrystalline cellulose (Vivapur 200) | | 15.7 |
| | Croscarmellose sodium | 3.0 | 3.0 |
| | Magnesium stearate | 0.5 | 0.5 |
| Total | | 75 | 75 |

### Experimental Example 3: Evaluation of content uniformity of final granules

The content uniformity of the final granules was evaluated for the tablets prepared in Comparative Example 4 and Example 5. The results are shown in Table 7 below. In this case, the content uniformity was evaluated in the same manner as in Experimental Example 2 above.

The content uniformity of the granules in the post-mixed part and the content uniformity of the final granules were tested for the tablets of Comparative Example 4 and Example 5 using the above method. As a result, it was confirmed that both the content in the final granules and the formulation uniformity (content uniformity between tablets) were excellent. However, as in Comparative Example 4, it was confirmed that the smaller the average particle size of microcrystalline cellulose, the weaker the granule flowability was. These results are summarized in detail in Table 7.

**[Table 7]**

| | | **Example 1** | **Comparative Example 4** | **Example 5** |
|---|---|---|---|---|
| Content uniformity of final granules | Average (%) | 96.3 | 102.1 | 102.2 |
| | SD | 0.7 | 1.9 | 1.7 |
| Formulation uniformity | Acceptance value (%) | 4.5 | 5.5 | 5.6 |
| Granule flowability | Carr's index (%) | 23.3 | 27.7 | 22.7 |

### Preparation of granular material according to ratio of excipients in pre-mixed granules and post-mixed part

In preparing a granular material in which pre-mixed granules and a post-mixed part were mixed, the most appropriate ratio of the diluent for the pre-mixed granules and the post-mixed part was searched for.

Tablets were prepared in the same manner as in Example 1, except that the ratios of microcrystalline cellulose used in the pre-mixed granules and the post-mixed part were used differently as shown in Table 8 below.

**[Table 8]**

| **Process** | **Additive** | **Example 6** | **Example 7** | **Comparative Example 5** |
|---|---|---|---|---|
| Pre-mixed granules | Enavogliflozin | 0.3 | 0.3 | 0.3 |
| | Microcrystalline cellulose (PH-102) | 10.0 | 32.9 | 55.7 |
| | Hydroxypropyl cellulose | 4.0 | 4.0 | 4.0 |
| | Light anhydrous silicic acid | 1.0 | 1.0 | 1.0 |
| | Magnesium stearate | 0.5 | 0.5 | 0.5 |
| Post-mixed part | Microcrystalline cellulose (Vivapur 12) | 55.7 | 32.8 | 10.0 |
| | Croscarmellose sodium | 3.0 | 3.0 | 3.0 |
| | Magnesium stearate | 0.5 | 0.5 | 0.5 |
| Total | | 75 | 75 | 75 |

### Experimental Example 4: Evaluation of flowability and content uniformity of final granules

The flowability and content uniformity of the final granules were evaluated for the tablets prepared in Example 6, Example 7, and Comparative Example 5. The results are shown in Table 9 below. In this case, the content uniformity was evaluated in the same manner as in Experimental Example 2 above.

The flowability of the final granules was tested for the tablets of Example 6, Example 7, and Comparative Example 5 using the above method. As a result, it can be seen that that the flowability of the granules became excellent as the proportion of the microcrystalline cellulose in the post-mixed part increased. In the case of content uniformity, it can be seen that the content deviation (SD) also increased as the proportion of the microcrystalline cellulose in the post-mixed part increased. This is summarized in detail in Table 9.

**[Table 9]**

| | | **Example 1** | **Example 6** | **Example 7** | **Comparative Example 5** |
|---|---|---|---|---|---|
| Granule flowability | Carr's index (%) | 23.3 | 20.6 | 22.5 | 26.0 |
| Content uniformity of final granules | Average (%) | 96.3 | 101.7 | 100.1 | 101.6 |
| | SD | 0.7 | 4.4 | 3.6 | 1.9 |
| Formulation uniformity | Acceptance value (%) | 4.5 | 13.8 | 11.4 | 5.3 |

### Preparation of tablets according to weight average molecular weight of hydroxypropyl cellulose

As shown in Table 10 below, tablets were prepared in the same manner as described in Example 1, except that hydroxypropyl cellulose having various weight average molecular weights was used as the binder.

**[Table 10]**

| | **Weight average molecular weight of hydroxypropyl cellulose** | **Type and amount (mg) of other raw materials** |
|---|---|---|
| Example 1 | 140,000 | Enavogliflozin 0.3 |
| Example 8 | 40,000 | Microcrystalline cellulose 65.7 |
| Comparative Example 6 | 700,000 | Croscarmellose 3.0 |
| | | Light anhydrous silicic acid 1.0 |
| | | Magnesium stearate 1.0 |

### Experimental Example 5: Dissolution test

A dissolution test was performed to compare the dissolution rates according to the weight average molecular weight of hydroxypropyl cellulose as the binder. The dissolution test was performed under the following conditions using pH 1.2 as the dissolution medium in consideration of the maximum absorption concentration time (Tₘₐₓ) of enavogliflozin and the solubility according to pH.
1) Dissolution method: Korean Pharmacopoeia Dissolution Method 2 (paddle method)
2) Dissolution medium: pH 1.2
3) Dissolution medium amount: 500 mL
4) Dissolution vessel temperature: 37.5°C ± 0.5°C
5) Paddle speed: 50 rpm
6) Analysis method: HPLC method
   - Detector: UV absorptiometer (Measurement wavelength: 225 nm)
   - Column: C18 5 µm/4.6 mm x 150 mm column
   - Mobile phase: hydrogen phosphate buffer + acetonitrile

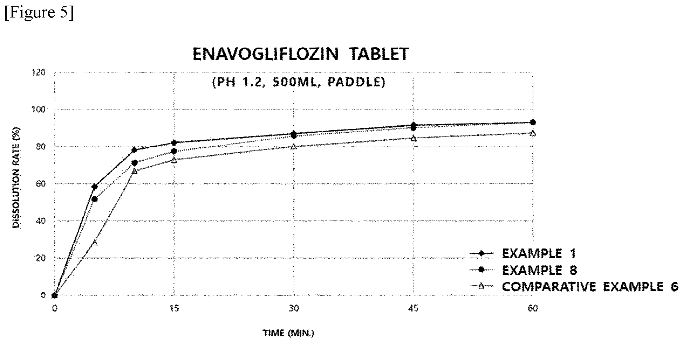

As a result, in the case of Comparative Example 6 using the binder, hydroxypropyl cellulose, having the highest weight average molecular weight of 700,000, as the initial dissolution rate was less than 30%, the dissolution rate was delayed, and the 30-minute dissolution rate also did not exceed 85%. Because enavogliflozin is a component having a Tₘₐₓ of approximately 1 hour, dissolution in gastric juice is expected to have a significant influence on bioavailability. Therefore, it was judged that the dissolution rate at pH 1.2 was particularly important, and the use of hydroxypropyl cellulose having a high weight average molecular weight of 200,000 or more as in Comparative Example 6 is not desirable in terms of bioavailability because it has a significant influence on the initial dissolution rate. The results of each dissolution rate evaluation are shown in detail in Table 11 below and FIG. 5.

**[Table 11]**

| | 5-minute dissolution rate (%) | | 15-minute dissolution rate (%) | | 30-minute dissolution rate (%) | |
|---|---|---|---|---|---|---|
| | Mean | Standard deviation | Mean | Standard deviation | Mean | Standard deviation |
| Example 1 | 58.5 | 2.9 | 82.1 | 2.7 | 87.0 | 2.1 |
| Example 8 | 51.7 | 2.2 | 77.4 | 2.8 | 85.8 | 3.2 |
| Comparative Example 6 | 28.4 | 3.1 | 72.8 | 4.0 | 80.1 | 4.8 |

## Claims

1. A pharmaceutical composition comprising a compound of Chemical Formula 1 or a pharmaceutically acceptable salt thereof, which serves as an active ingredient, an excipient, a disintegrant, and a binder, wherein the compound of Chemical Formula 1 has an average particle size of 15 µm or less.

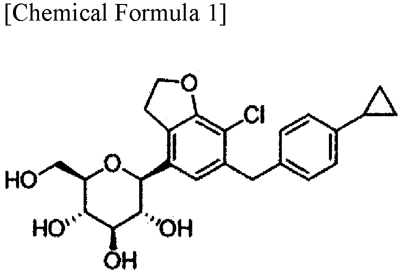

2. The pharmaceutical composition of claim 1, wherein the compound of Chemical Formula 1 is included in an amount of less than 1 part by weight based on 100 parts by weight of the total pharmaceutical composition.

3. The pharmaceutical composition of claim 1, wherein the content of the active ingredient in the pharmaceutical composition ranges from 0.1 to 0.5 mg.

4. The pharmaceutical composition of claim 1, wherein the excipient is included in an amount of 80 to 95 parts by weight based on 100 parts by weight of the total pharmaceutical composition.

5. The pharmaceutical composition of claim 1, wherein the disintegrant is included in an amount of 2 to 8 parts by weight based on 100 parts by weight of the total pharmaceutical composition.

6. The pharmaceutical composition of claim 1, wherein the binder is included in an amount of 3 to 10 parts by weight based on 100 parts by weight of the total pharmaceutical composition.

7. The pharmaceutical composition of claim 1, wherein a dissolution rate of the pharmaceutical composition is 50% or more of the total content of the active ingredient after 5 minutes.

8. The pharmaceutical composition of claim 1, wherein the dissolution rate of the pharmaceutical composition is 80% or more of the total content of the active ingredient after 15 minutes.

9. The pharmaceutical composition of claim 1, wherein the dissolution rate of the pharmaceutical composition is 85% or more of the total content of the active ingredient after 30 minutes.

10. A pharmaceutical composition comprising a granular material in which pre-mixed granules and a post-mixed part are mixed, wherein the pre-mixed granules include a compound of Chemical Formula 1 or a pharmaceutically acceptable salt thereof.

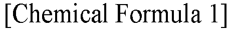

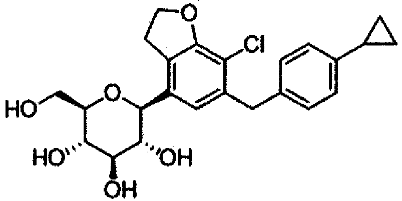

11. The pharmaceutical composition of claim 10, wherein the pre-mixed granules include the compound of Chemical Formula 1 or a pharmaceutically acceptable salt thereof, an excipient, a binder, and a lubricant.

12. The pharmaceutical composition of claim 10, wherein the post-mixed part includes an excipient, a disintegrant, and a lubricant.

13. The pharmaceutical composition of claim 10, wherein each of the pre-mixed granules and the post-mixed part includes an excipient.

14. The pharmaceutical composition of claim 13, wherein each of the pre-mixed granules and the post-mixed part includes microcrystalline cellulose as the excipient.

15. The pharmaceutical composition of claim 14, wherein the microcrystalline cellulose in the pre-mixed granules has a particle size of 130 µm or less.

16. The pharmaceutical composition of claim 14, wherein the microcrystalline cellulose in the pre-mixed granules has a bulk density of 0.26 to 0.33.

17. The pharmaceutical composition of claim 14, wherein the microcrystalline cellulose in the post-mixed part has a particle size of 130 µm or more.

18. The pharmaceutical composition of claim 14, wherein the excipient in the post-mixed part has a bulk density of 0.28 to 0.37.

19. The pharmaceutical composition of claim 13, wherein a weight ratio of the excipient in the pre-mixed granules and the excipient in the post-mixed part ranges from 4:1 to 1:1.

20. The pharmaceutical composition of claim 11, wherein the binder is one or more selected from the group consisting of hydroxypropyl cellulose, povidone, copovidone, and hypromellose.

21. The pharmaceutical composition of claim 20, wherein the binder is hydroxypropyl cellulose, which has a weight average molecular weight of less than 200,000.

22. The pharmaceutical composition of claim 10, wherein the granular material has a Carr's index of 21 to 25.

23. The pharmaceutical composition of claim 10, wherein the granular material is a dry granular material.

24. The pharmaceutical composition of claim 10, wherein the pharmaceutical composition is in the form of a tablet.

25. The pharmaceutical composition of claim 10, wherein the pharmaceutical composition includes the compound of Chemical Formula 1 in a dose of 0.3 mg.
